# FASCICULE DE BREVET EUROPEEN

(11) **EP 1 859 784 B1**
(45) Date de publication et mention de la délivrance du brevet: **16.01.2013**
(21) Numéro de dépôt: 07108285.3
(22) Date de dépôt: 15.05.2007
(51) Int. Cl.: A61K 8/49, A61K 8/41, A61K 8/97, A61Q 5/06

(54) **Utilisation d`un hydrotrope cationique pour la coloration de fibres kératiniques, composition le comprenant et procédés de coloration la mettant en oeuvre**
Verwendung eines kationischen Hydrotrops zur direkten Färbung keratinischer Fasern, Zusammensetzung dieses enthaltend und Verfahren zu Haarfärbung
Use of a cationic hydrotrope for direct dyeing of keratin fibres, composition containing it and hair dyeing method

(30) Priorité: 22.05.2006 FR 0651871
(43) Date de publication de la demande: 28.11.2007
(73) Titulaire: L'Oréal, 75008 Paris (FR)
(72) Inventeur: De Boni, Maxime, 75020, Paris (FR); Lagrange, Alain, 77700, Coupvary (FR)
(74) Mandataire: Fevrier, Murielle Françoise E.

(56) Documents cités:
- EP-A- 1 627 625
- WO-A-03/030842
- WO-A-2006/131234
- US-A- 2 848 369

## Description

La présente invention a pour objet l'utilisation d'un agent hydrotrope cationique pour la coloration directe des fibres kératiniques. Elle concerne de même une composition tinctoriale comprenant au moins un colorant et un tel hydrotrope cationique, ainsi que des procédés de coloration mettant en oeuvre cette composition tinctoriale.

Dans le domaine de la coloration des fibres kératiniques, notamment humaines, telles que les cheveux, on a développé deux modes de coloration.
Le premier mode, appelé aussi coloration permanente, consiste à appliquer sur les fibres kératiniques, des compositions contenant des précurseurs de colorant d'oxydation, appelés généralement des bases d'oxydation, tels que par exemple les ortho- ou paraphénylènediamines, les ortho- ou para-aminophénols ou encore des composés hétérocycliques. Ces précurseurs de colorants d'oxydation sont des composés incolores ou faiblement colorés qui, associés à des produits oxydants, permettent d'accéder, par un processus de condensation oxydative, à des composés colorés. Ces composés colorés, insolubles dans le milieu de teinture, sont piégés à l'intérieur du cheveu.
On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques tels que des composés indoliques.
Le deuxième mode de coloration, appelé aussi coloration directe ou semi-permanente, consiste à appliquer des compositions comprenant des colorants directs qui sont des molécules colorées et colorantes ayant une affinité pour les fibres et qui pénètrent par diffusion, à l'intérieur de celles-ci. Il est connu par exemple d'utiliser des colorants directs nitrés benzéniques, anthraquinoniques, nitropyridiniques, azoïques, xanthéniques, acridiniques, aziniques ou triarylméthaniques.
Contrairement aux compositions de teinture par oxydation, les compositions de teinture directe peuvent avantageusement être mises en oeuvre sans la présence obligatoire d'un agent oxydant, à moins que l'on ne souhaite avoir, avec la coloration des fibres, un effet d'éclaircissement de celles-ci.
On obtient avec la coloration directe, des colorations souvent plus chromatiques qu'avec la coloration d'oxydation, mais qui sont cependant temporaires ou semi-permanentes. En effet, à cause de la nature des liaisons existant entre les colorants directs et la fibre kératinique, leur désorption de la surface et/ou du coeur de la fibre se fait relativement facilement.

Il est évidemment possible de mettre en oeuvre des colorations comprenant à la fois des précurseurs de colorant d'oxydation et des colorants directs.

De très nombreuses compositions de coloration ont été proposées à ce jour qui donnent satisfaction notamment en terme de variété de couleurs et de reflets. Cependant, d'une part, les colorations obtenues manquent parfois de puissance et/ou d'homogénéité. D'autre part, on continue de rencontrer encore des problèmes de mise en oeuvre des colorants. En effet, certains colorants ou précurseurs de colorants peuvent avoir une limite de solubilité peu élevée dans les compositions tinctoriales conventionnelles, avec pour conséquence, entre autres, de voir diminuer l'effet de coloration recherché. En outre, ce critère de solubilité réduit le nombre de colorants qui peuvent être utilisés.
Une solution pour remédier à cet inconvénient serait de limiter la quantité en ce ou ces colorants dans la composition. Mais dans ce cas, les problèmes de baisse d'efficacité de la coloration comme par exemple une insuffisance de la puissance de la couleur ou de la couverture des cheveux blancs, une sélectivité (différence de couleur entre les parties d'une même fibre et/ou entre plusieurs fibres) trop importante, ne seraient toujours pas résolus de manière satisfaisante.
La présente invention a donc pour objet de résoudre les problèmes mentionnés ci-dessus.

Ces buts et d'autres sont atteints par la présente invention qui a donc pour objet l'utilisation d'au moins un colorant directe et d'au moins un hydrotrope cationique de formule (I) suivante : 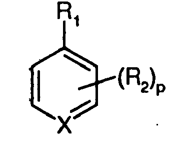 Dans laquelle :
- X représentant N⁺R₆ avec R₆ représentant un radical alkyle en C₁-C₁₀ linéaire ou ramifié;
- R₁ représente :
   - un atome d'hydrogène, un radical alkyle C₁-C₁₀ linéaire ou ramifié
- R₂ représente un radical alkyle C₁-C₄ linéaire ou ramifié;
- p vaut 0 ou 1 ;
- l'électroneutralité du composé étant assuré par un anion ou un mélange d'anions cosmétiquement aceptables,
   pour la coloration des fibres kératiniques.

Elle a de même pour objet une composition tinctoriale comprenant, dans un milieu approprié pour la coloration, au moins un hydrotrope cationique tel que défini précédemment et au moins un colorant direct.

Elle a également pour objet un procédé de coloration de fibres kératiniques, dans lequel on met en oeuvre les étapes suivantes :
a) On applique sur les fibres kératiniques la composition tinctoriale précitée ;
b) On laisse poser pendant une durée suffisante pour obtenir l'effet souhaité,
c) On rince éventuellement les fibres kératiniques ;
d) On lave au shampooing et on rince les fibres, puis on les sèche ou on les laisse sécher.

Elle concerne aussi un procédé de coloration dans lequel on met en oeuvre les étapes décrites auparavant, à l'exception du fait que l'étape a) consiste à appliquer simultanément ou successivement sur les fibres kératiniques, d'une part ladite composition tinctoriale, et d'autre part une composition oxydante comprenant au moins un agent oxydant.

Elle concerne enfin un procédé de coloration de fibres kératiniques, dans lequel on met en oeuvre les étapes décrites auparavant, à l'exception du fait que la composition appliquée sur les fibres est une composition prête à l'emploi obtenue en mélangeant une composition colorante avec une composition comprenant au moins un agent oxydant ; l'agent hydrotrope cationique se trouvant dans la composition colorante, dans la composition comprenant l'agent oxydant ou dans ces deux compositions simultanément.

Mais d'autres caractéristiques et avantages de la présente invention apparaîtront plus clairement à la lecture de la description et des exemples qui vont être présentés.

Dans ce qui va suivre et à moins qu'une autre indication ne soit donnée, les bornes délimitant un domaine de valeurs sont comprises dans ce domaine.
Par fibres kératiniques, on entend plus particulièrement des fibres kératiniques humaines, comme les cils, les sourcils et les cheveux. De préférence, les fibres kératiniques humaines sont des cheveux.

Comme cela a été indiqué auparavant, l'agent hydrotrope cationique employé dans le cadre de la présente invention correspond à la formule (I) ci-dessous : 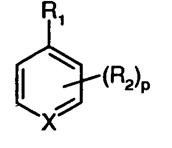 Dans laquelle :
- X représentant N⁺R₆ avec R₆ représentant un radical alkyle en C₁-C₁₀ linéaire ou ramifié, de préférence en C₁-C₆
- R₁ représente :
   - un atome d'hydrogène, un radical alkyle C₁-C₁₀ linéaire ou ramifié, de préférence en C₁-C₆,
- R₂ représente un radical alkyle C₁-C₄ linéaire ou ramifié ;
- p vaut 0 ou 1 ;
- l'électroneutralité du composé étant assuré par un anion ou un mélange d'anions cosmétiquement aceptables.

Plus particulièrement, l'anion ou le mélange d'anions cosmétiquement acceptables est choisi parmi halogénures, comme les chlorures, les bromures ; des sulfates ; des alkylsulfates pour lesquels la partie alkyle, linéaire ou ramifiée, est en C₁-C₆, comme les ions méthosulfates, éthosulfates ; des hydrogénocarbonates ; des perchlorates ; des sels d'acides carboxyliques comme les acétates, les citrates, les tartrates, seuls ou combinés.

A titre d'exemples d'hydrotropes cationiques préférés, on peut citer sans intention de s'y limiter :

| | |
|---|---|
| Sel de N-butyl-3,4-diméthylpyridinium | 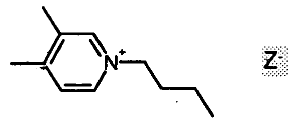 |
| Sel de N-butyl-4-méthylpyridinium | 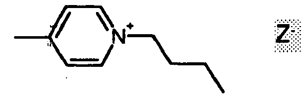 |

Dans les formules ci-dessus, Z⁻ représente un anion choisi parmi ceux mentionnés auparavant. De préférence, Z⁻ représente un ion chlorure.

Un autre objet de la présente invention est constitué par une composition tinctoriale comprenant, dans un milieu approprié pour la coloration, au moins un hydrotrope cationique tel qu'il vient d'être défini, ainsi qu'au moins un colorant direct.

Plus particulièrement, la composition tinctoriale comprend de 0,1 à 50 % en poids d'agent hydrotrope cationique par rapport au poids de la composition tinctoriale, plus particulièrement de 0,1 à 20 % en poids et de préférence de 1 à 15 % en poids par rapport au poids de la composition tinctoriale.

Les colorants directs utilisables sont choisis parmi les espèces ioniques ou non ioniques.
A titre d'exemples non limitatifs, on peut citer les colorants benzéniques nitrés, les colorants azoïques, azométhiniques, méthiniques, tétraazapenthaméthiniques, anthraquinoniques, naphtoquinoniques, benzoquinoniques, phénotiaziniques indigoïdes, xanthéniques, phénanthridiniques, phtalocyanines, ceux dérivés du triarylméthane et les colorants naturels, seuls ou en mélanges.
Il peut par exemple être choisi parmi les colorants benzéniques nitrés rouges ou orangés suivants :
- le 1-hydroxy-3-nitro-4-N-(γ-hydroxypropyl)amino benzène,
- le N-(β-hydroxyéthyl)amino-3-nitro-4-amino benzène,
- le 1-amino-3-méthyl-4-N-(β-hydroxyéthyl)amino-6-nitro benzène,
- le 1-hydroxy-3-nitro-4-N-(β-hydroxyéthyl)amino benzène,
- le 1,4-diamino-2-nitrobenzène,
- le 1-amino-2-nitro-4-méthylamino benzène,
- la N-(β-hydroxyéthyl)-2-nitro-paraphénylènediamine,
- le 1-amino-2-nitro-4-(β-hydroxyéthyl)amino-5-chloro benzène,
- la 2-nitro-4-amino-diphénylamine,
- le1-amino-3-nitro-6-hydroxybenzène.
- le 1-(β-aminoéthyl)amino-2-nitro-4-(β-hydroxyéthyloxy) benzène,
- le 1-(β, γ-dihydroxypropyl)oxy-3-nitro-4-(β-hydroxyéthyl)amino benzène,
- le 1-hydroxy-3-nitro-4-aminobenzène,
- le 1-hydroxy-2-amino-4,6-dinitrobenzène,
- le 1-méthoxy-3-nitro-4-(β-hydroxyéthyl)amino benzène,
- la 2-nitro-4'-hydroxydiphénylamine,
- le 1-amino-2-nitro-4-hydroxy-5-méthylbenzène,
- le 2-[4-(2-chloro-4-nitrophénylazo)-N-éthylphénylamino]éthanol (Disperse Red 13).
   Le colorant direct peut aussi être choisi parmi les colorants directs benzéniques nitrés jaunes et jaune-verts, on peut par exemple citer les composés choisis parmi:

- le 1-β-hydroxyéthyloxy-3-méthylamino-4-nitrobenzène,
- le 1-méthytamino-2-nitro-5-(β,γ-dihydroxypropyt)oxy benzène,
- le 1-(β-hydroxyéthyl)amino-2-méthoxy-4-nitrobenzène,
- le 1-(β-aminoéthyl)amino-2-nitro-5-méthoxy-benzène,
- le 1,3-di(β-hydroxyéthyl)amino-4-nitro-6-chlorobenzène,
- le 1-amino-2-nitro-6-méthyl-benzène,
- le 1-(β-hydroxyéthyl)amino-2-hydroxy-4-nitrobenzène,
- la N-(β-hydroxyéthyl)-2-nitro-4-trifluorométhylaniline,
- l'acide 4-(β-hydroxyéthyl)amino-3-nitro-benzènesulfonique,
- l'acide 4-éthylamino-3-nitro-benzoïque,
- le 4-(β-hydroxyéthyl)amino-3-nitro-chlorobenzène,
- le 4-(β-hydroxyéthyl)amino-3-nitro-méthylbenzène,
- le 4-(β,γ-dihydroxypropyl)amino-3-nitro-trifluorométhylbenzène,
- le 1-(β-uréidoéthyl)amino-4-nitrobenzène,
- le 1,3-diamino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzène,
- le 4-(β-hydroxyéthyl)amino-3-nitrobenzamide.
   On peut aussi mentionner les colorants directs benzéniques nitrés bleus ou violets, comme par exemple :

- le 1-(β-hydroxyéthyl)amino-4-N,N-bis-(β-hydroxyéthyl)amino 2-nitrobenzène,
- le 1-(γ-hydroxypropyl)amino 4-N,N-bis-(β-hydroxyéthyl)amino 2-nitrobenzène,
- le 1-(β-hydroxyéthyl)amino 4-(N-méthyl, N-β-hydroxyéthyl)amino 2-nitrobenzène,
- le 1-(β-hydroxyéthyl)amino 4-(N-éthyl, N-β-hydroxyéthyl)amino 2-nitrobenzène,
- le 1-(β,γ-dihydroxypropyl)amino 4-(N-éthyl, N-β-hydroxyéthyl)amino 2-nitrobenzène,
- les 2-nitroparaphénylènediamines de formule suivante : 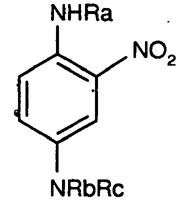
dans laquelle :
- Rb représente un radical alkyle en C₁-C₄, un radical β-hydroxyéthyle ou β -hydroxypropyle ou γ-hydroxypropyle ;
- Ra et Rc, identiques ou différents, représentent un radical β-hydroxyéthyle, -β-hydroxypropyle, γ-hydroxypropyle, ou β,γ-dihydroxypropyle, l'un au moins des radicaux Rb, Rc ou Ra représentant un radical γ-hydroxypropyle et Rb et Rc ne pouvant désigner simultanément un radical β-hydroxyéthyle lorsque Rb est un radical γ-hydroxypropyle, telles que celles décrits dans le brevet français FR 2 692 572.

Parmi les colorants directs azoïques utilisables selon l'invention on peut aussi citer les colorants azoïques cationiques décrits dans les demandes de brevets WO 95/15144, WO 95/01772, EP 714954, FR 2 822 696, FR 2 825 702, FR 2 825 625, FR 2 822 698, FR 2 822 693, FR 2 822 694, FR 2 829 926, FR 2 807 650, WO 02/078660, WO 02/100834, WO 02/100369, FR 2 844 269.
Parmi ces composés on peut tout particulièrement citer les colorants suivants :
- chlorure de 1,3-diméthyl-2-[[4-(diméthylamino)phényl]azo]-1 H-Imidazolium,
- chlorure de 1,3-diméthyl-2-[(4-aminophényl)azo]-1 H-Imidazolium,
- méthylsulfate de 1-méthyl-4-[(méthylphénylhydrazono)méthyl]-pyridinium.
   On peut également citer parmi les colorants directs azoïques les colorants suivants, décrits dans le COLOUR INDEX INTERNATIONAL 3e édition : Disperse Red 17, Acid Yellow 9, Acid Black 1, Basic Red 22, Basic Red 76, Basic Yellow 57, Basic Brown 16, Acid Yellow 36, Acid Orange 7, Acid Red 33, Acid Red 35, Basic Brown 17, Acid Yellow 23, Acid Orange 24, Disperse Black 9.
   On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.
   Parmi les colorants directs quinoniques on peut citer les colorants suivants : Disperse Red 15, Solvent Violet 13, Acid Violet 43, Disperse Violet 1, Disperse Violet 4, Disperse Blue 1, Disperse Violet 8, Disperse Blue 3, Disperse Red 11, Acid Blue 62, Disperse Blue 7, Basic Blue 22, Disperse Violet 15, Basic Blue 99, ainsi que les composés suivants:

- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.
Parmi les colorants aziniques on peut citer les composés suivants : Basic Blue 17, Basic Red 2.
Parmi les colorants triarylméthaniques utilisables selon l'invention, on peut citer les composés suivants : Basic Green 1, Acid blue 9, Basic Violet 3, Basic Violet 14, Basic Blue 7, Acid Violet 49, Basic Blue 26, Acid Blue 7.
Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.
   Parmi les colorants de type tétraazapentaméthiniques utilisables selon l'invention, on peut citer notamment le chlorure de 2-((E)-{(E)-[(1,3-dimethyl-1,3-dihydro-2H-imidazol-2-ylidene)hydrazono]methyl}diazenyl)-1,3-dimethyl-1H-imidazol-3-ium, le chlorure de 2-{(E)-[(1Z)-N-(1,3-dimethyl-1,3-dihydro-2H-imidazol-2-ylidene)ethane hydrazonoyl] diazenyl}-1,3-dimethyl-1H-imidazol-3-ium, le chlorure de 4-methoxy-2-((E)-{(1E)-1-[(2E)-(4-methoxy-1-methylpyridin-2(1H)-ylidene)hydrazono]ethyl}diazenyl)-1-methylpyridinium, le chlorure de 1-methyl-2-((E)-{(1E)-1-[(2E)-(1-methylpyridin-2(1H)-ylidene)hydrazono]ethyl} diazenyl) pyridinium, le chlorure de 1-(2-hydroxyethyl)-2-[(E)-((1E)-1-{(2E)-[1-(2-hydroxyethyl)pyridin-2(1H)-ylidene]hydrazono}ethyl)diazenyl]pyridinium, le chlorure de 1-methyl-2-((E)-{(E)-[(2Z)-(1-methylpyridin-2(1H)-ylidene)hydrazono]methyl}diazenyl)pyridinium, l'acétate de 1-(2-hydroxyethyl)-2-[(E)-{(E)-((2E)-[1-(2-hydroxyethyl)pyridin-2(1 H)-ylidene]hydrazono}methyl) diazenyl]pyridinium.
   Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.
   Les colorants azoiques sont particulièrement utiles dans la présente invention.

La teneur en colorant(s) direct(s) dans la composition tinctoriale varie en général de 0,001 à 20% en poids par rapport au poids de la composition tinctoriale, et de préférence de 0,01 à 10% en poids.

La composition de l'invention peut aussi comprendre des colorants d'oxydation, ces derniers pouvant être choisis parmi les bases d'oxydation, les coupleurs, ou leurs mélanges.
Plus particulièrement, les bases d'oxydation peuvent être choisies parmi les bases d'oxydation classiquement utilisées en teinture d'oxydation, par exemple les paraphénylènediamines, les bis-phénylalkylènediamines, les para-aminophénols, les ortho-aminophénols et les bases hétérocycliques.
Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β -hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, la 4 aminophenyl pyrrolidine, le 2 thiényl paraphénylène diamine, le 2-β hydroxyéthylamino 5-amino toluène et leurs sels d'addition avec un acide.
Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.
Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.
Parmi les para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(P-hydroxyéthyl aminométhyl) phénol, le 4-amino 2-fluoro phénol, et leurs sels d'addition avec un acide.
Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.
Parmi les bases hétérocycliques, on peut citer à titre d'exemple, les dérivés pyridiniques, les dérivés pyrimidiniques et les dérivés pyrazoliques.
Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.
Parmi les dérivés pyrimidiniques, on peut citer les composés décrits par exemple dans les brevets DE 2 359 399 ; JP 88-169 571 ; JP 05 163 124 ; EP 0 770 375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxyéthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.
Parmi les dérivés pyrazoliques, on peut citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.
Lorsque la composition tinctoriale comprend une ou plusieurs bases d'oxydation, leur teneur est habituellement comprise entre 0,001 et 10 % en poids du poids de la composition tinctoriale, et de préférence entre 0,005 et 6 % en poids.

La composition de l'invention peut contenir des coupleurs, ces derniers peuvent être choisis parmi ceux conventionnellement utilisés pour la coloration de fibres kératiniques.
On peut notamment citer les métaphénylènediamines, les méta-aminophénols, les métadiphénols, les coupleurs naphtaléniques et les coupleurs hétérocycliques.
A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition avec un acide.
Si la composition comprend un ou plusieurs coupleurs, leur teneur représente classiquement de 0,001 à 10 % en poids du poids de la composition tinctoriale, et de préférence de 0,005 à 6 % en poids.

D'une manière générale, les sels d'addition avec un acide sont choisis par exemple parmi les chlorhydrates, les bromhydrates, les sulfates, les citrates, les succinates, les tartrates, les lactates, les tosylates, les benzènesulfonates, les phosphates et les acétates.

Il est par ailleurs précisé que la composition tinctoriale ne comprend pas d'agent oxydant.

Le milieu approprié pour la coloration comprend généralement de l'eau ou un mélange d'eau et d'au moins un solvant organique pour solubiliser les composés qui ne seraient pas suffisamment solubles dans l'eau.
Plus particulièrement, les solvants organiques sont choisis parmi les monoalcools ou les diols, linéaires ou ramifiés, de préférence saturés, comprenant 2 à 10 atomes de carbone, tels que l'alcool éthylique, l'alcool isopropylique, l'hexylèneglycol (2-méthyl 2,4-pentanediol), le néopentylglycol et le 3-méthyl-1,5-pentanediol ; les alcools aromatiques tels que l'alcool benzylique, l'alcool phényléthylique ; les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ; ainsi que les alkyléthers de diéthylèneglycol, notamment en C₁-C₄, comme par exemple, le monoéthyléther ou le monobutyléther du diéthylèneglycol, seuls ou en mélange.
Les solvants usuels décrits ci-dessus, s'ils sont présents, représentent habituellement de 1 à 40 % en poids, de préférence de 5 à 30 % en poids, par rapport au poids de la composition tinctoriale.

Selon une variante avantageuse de l'invention, la composition tinctoriale comprend au moins 40 % en poids d'eau par rapport au poids de la composition tinctoriale, de préférence au moins 70 % en poids d'eau.

La composition tinctoriale mise en oeuvre dans le cadre de l'invention peut également renfermer divers adjuvants utilisés classiquement dans les compositions pour la teinture des fibres kératiniques, tels que des agents tensioactifs anioniques, cationiques, non ioniques, amphotères, zwittérioniques ou leurs mélanges, des polymères anioniques, cationiques, non ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.
De préférence, la composition tinctoriale comprend au moins un agent tensioactif, un polymère, ou leurs mélanges.

Les adjuvants ci dessus sont en général présents en quantité comprise pour chacun d'eux entre 0,01 et 20 % en poids par rapport au poids de la composition tinctoriale.

Bien entendu, l'homme de l'art veillera à choisir ce ou ces éventuels adjuvants de manière telle que les propriétés avantageuses attachées intrinsèquement à la composition tinctoriale mise en oeuvre dans le cadre de l'invention ne soient pas, ou substantiellement pas, altérées par la ou les adjonctions envisagées.

Le pH de la composition tinctoriale est généralement compris entre 2 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.
Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.
Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule suivante : 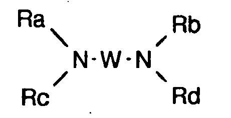 dans laquelle W est un reste propylène éventuellement substitué par un groupement hydroxyle ou un radical alkyle en C₁-C₄ ; Rₐ, R_{b}, P_{c} et R_{d}, identiques ou différents, représentent un atome d'hydrogène, un radical alkyle en C₁-C₄ ou hydroxyalkyle en C₁-C₄.

Il est à noter que la composition tinctoriale peut se présenter sous des formes diverses, telles que sous forme de liquides, de crèmes, de gels, de pâtes ou sous toute autre forme appropriée pour réaliser une coloration des fibres kératiniques, et notamment des cheveux humains.

Un autre objet selon l'invention concerne un procédé de coloration de fibres kératiniques dans lequel on met en oeuvre les étapes suivantes :
a) on applique sur lesdites fibres, une composition tinctoriale telle qu'elle vient d'être détaillée ;
b) on laisse poser ladite composition pendant une durée suffisante pour obtenir l'effet souhaité,
c) on rince éventuellement les fibres kératiniques;
d) on lave au shampooing et on rince les fibres, puis on les sèche ou on les laisse sécher.

Cette variante est particulièrement appropriée dans le cas où la composition tinctoriale ne comprend pas de colorant d'oxydation.

Un autre objet de l'invention est constitué par un procédé consistant à mettre en oeuvre les étapes suivantes :
a) On applique simultanément ou successivement sur les fibres kératiniques, d'une part, une composition tinctoriale telle que définie précédemment, et d'autre part, une composition oxydante comprenant au moins un agent oxydant ;
b) On laisse poser pendant une durée suffisante pour obtenir l'effet souhaité,
c) On rince éventuellement les fibres kératiniques ;
d) On lave au shampooing et on rince les fibres, puis on les sèche ou on les laisse sécher.

Il est à noter que cette variante de procédé est appropriée si la composition tinctoriale comprend au moins un précurseur de colorant comme les bases d'oxydation et les coupleurs, ou encore si la composition comprend un ou plusieurs colorants directs et que l'on souhaite obtenir un effet d'éclaircissement des fibres kératiniques à traiter.
La composition oxydante mélangée à la composition tinctoriale comprend au moins un agent oxydant choisi parmi ceux employés classiquement dans ce domaine.
Ces derniers sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydoréductases à 2 électrons telles que les uricases et les oxygénases à 4 électrons comme les laccases.
Selon un mode de réalisation particulier, la composition contient un agent oxydant de type peroxyde et/ou un agent oxydant de type persels, par exemple un mélange peroxyde d'hydrogène et persulfates ou le peroxyde d'hydrogène seul.
La teneur en agent oxydant est en général comprise entre 1 et 60 % en poids, par rapport au poids de la composition prête à l'emploi de préférence entre 1 et 40 % en poids par rapport au poids de la composition prête à l'emploi.
La composition oxydante peut également renfermer divers adjuvants utilisés classiquement dans les compositions pour la teinture des fibres kératiniques et tels que définis précédemment dans le cadre de la description de la composition tinctoriale.

Le pH de la composition oxydante est tel qu'après mélange avec la composition tinctoriale, le pH de la composition prête à l'emploi appliquée sur les fibres kératiniques varie avantageusement entre 4 et 12 environ, et de préférence entre 7 et 11,5. Il peut être ajusté à la valeur désirée au moyen d'agents acidifiants ou alcalinisants habituellement utilisés en teinture des fibres kératiniques et tels que définis précédemment.
Généralement, la composition oxydante utilisée est une composition aqueuse et peut se trouver sous la forme d'une solution ou encore d'une émulsion.
Habituellement, on mélange la composition tinctoriale avec environ 0,5 à 10 équivalents en poids de la composition oxydante.

Un dernier objet de l'invention est constitué par un procédé de coloration de fibres kératiniques, dans lequel on met en oeuvre les étapes suivantes :
a) On prépare une composition prête à l'emploi en mélangeant une composition colorante comprenant dans un milieu approprié à la coloration, au moins un colorant ou un précurseur de colorant, avec une composition oxydante comprenant au moins un agent oxydant ; l'agent hydrotrope cationique se trouvant dans la composition colorante, dans la composition comprenant l'agent oxydant ou dans ces deux compositions simultanément,
b) On applique sur les fibres kératiniques, la composition prête à l'emploi ainsi obtenue et on laisse poser pendant une durée suffisante pour obtenir l'effet souhaité,
c) on rince éventuellement les fibres kératiniques ;
d) on lave au shampooing et on rince les fibres, puis on les sèche ou on les laisse sécher.

Ce procédé est particulièrement approprié si la composition appliquée sur les fibres comprend au moins un précurseur de colorant comme les bases d'oxydation et les coupleurs, ou encore si la composition comprend un ou plusieurs colorants directs et que l'on souhaite obtenir un effet d'éclaircissement des fibres kératiniques à traiter.
De préférence, la composition comprend au moins un colorant direct.

La composition colorante mise en oeuvre dans ce procédé correspond à la composition tinctoriale qui a été décrite auparavant, à l'exception du fait qu'elle ne comprend pas l'hydrotrope cationique.
Selon une première variante, l'hydrotrope cationique est présent dans la composition colorante. Dans ce cas, la composition colorante présente les caractéristiques de la composition tinctoriale décrite auparavant.
Selon une deuxième variante, l'hydrotrope est présent dans la composition oxydante. Dans ce cas, sa teneur est telle qu'elle rentre dans les gammes de concentrations données plus haut, exprimées par rapport au poids de la composition tinctoriale, c'est-à-dire par rapport au poids de la composition colorante et de l'hydrotrope cationique.
La troisième variante représentant une combinaison des deux précédentes.

Ce qui a été indiqué auparavant concernant la composition tinctoriale et la composition oxydante reste valable et l'on pourra s'y reporter pour plus de détail.

Selon ce procédé, les composition colorante (ou tinctoriale) et oxydante sont appliquées simultanément ou successivement, avec ou sans rinçage intermédiaire.

Selon le procédé mis en oeuvre, la composition tinctoriale, la composition prête à l'emploi, et éventuellement la composition oxydante, sont appliquées sur les fibres kératiniques, sèches ou humides, puis laissées pendant un temps de pose suffisant pour obtenir la coloration recherchée.
Quelle que soit la variante retenue (avec ou sans agent oxydant) le temps de pose est en général compris entre quelques secondes et une heure, de préférence entre 3 et 30 minutes.
Dans le cas où les compositions tinctoriales et oxydantes sont appliquées successivement, le temps de pose de chacune d'entre elle varie dans les mêmes gammes que celles indiquées ci-dessus.

La température à laquelle la composition est laissée agir, est en général comprise entre 15 et 220°C, plus particulièrement entre 15 et 80°C, de préférence entre 15 et 40 °C.

A l'issue du temps de pose, la composition appliquée est éliminée par un rinçage à l'eau, suivi éventuellement d'un lavage avec un shampooing, puis éventuellement d'un séchage.

Les exemples suivants illustrent l'invention sans toutefois en limiter la portée.

### Exemple 1

On prépare une composition tinctoriale comprenant le colorant Disperse Red 13 a été à 0.5% dans un mélange constitué de 15% d'éthanol, de 5% d'alcool benzylique, de 2% de chlorure de N-butyl-3,4-diméthylpyridnium et de 78 % d'eau (pourcentages exprimés en poids).

On applique la composition sur une mèche de cheveux contenant 90% de cheveux naturels ainsi que sur une mèche de cheveux contenant 90% de cheveux permanentés, et on laisse poser pendant 20 minutes.
Après rinçage et séchage, les mèches sont colorées en rouge de façon puissante et homogène.

## Revendications

1. Utilisation d'au moins un colorant direct et d'au moins un hydrotrope cationique de formule (I) suivante : 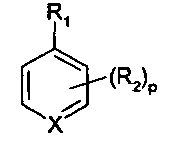 Dans laquelle :
• X représentant N⁺R₆ avec R₆ représentant un radical alkyle en C₁-C₁₀ linéaire ou ramifié, de préférence en C₁-C₆
• R₁ représente :
- un atome d'hydrogène, un radical alkyle C₁-C₁₀ linéaire ou ramifié, de préférence en C₁-C₆
• R₂ représente un radical alkyle C₁-C₄ linéaire ou ramifié ;
• p vaut 0 ou 1 ;
• l'électroneutralité du composé étant assuré par un anion ou un mélange d'anions cosmétiquement aceptables,
pour la coloration des fibres kératiniques.

2. Utilisation selon la revendication 1, **caractérisé en ce que** l'anion ou le mélange d'anions cosmétiquement acceptables est choisi parmi halogénures ; des sulfates ; des alkylsulfates pour lesquels la partie alkyle, linéaire ou ramifiée, est en C₁-C₆, comme les ions méthosulfates, éthosulfates ; des hydrogénocarbonates ; des perchlorates ; des sels d'acides carboxyliques comme les acétates, les citrates, les tartrates, seuls ou combinés.

3. Utilisation selon l'une quelconques des revendications 1 et 2, **caractérisé en ce que** l'hydrotrope cationique est choisi parmi les sels de N-butyl-3,4-diméthylpyridinium, N-butyl-4-méthylpyridinium, seuls ou en mélanges.

4. Composition tinctoriale pour la coloration des fibres kératiniques humaines, comprenant dans un milieu approprié pour la coloration, au moins un hydrotrope cationique tel que défini à l'une des revendications 1 à 3, et au moins un colorant direct.

5. Composition tinctoriale selon la revendication précédente, **caractérisée en ce que** la teneur en hydrotrope cationique est comprise entre 0,5 et 50 % en poids par rapport au poids de la composition tinctoriale, de préférence entre 0,5 et 20 % en poids et de manière encore plus préférée de 1 à 15 % en poids.

6. Composition tinctoriale selon l'une quelconque des revendications 4 ou 5, **caractérisée en ce que** le colorant direct est choisi parmi les colorants directs ioniques ou non ioniques, notamment du type des colorants benzéniques nitrés, les colorants azoïques, azométhiniques, méthiniques, tétraazapenthaméthiniques, anthraquinoniques, naphtoquinoniques, benzoquinoniques, phénotiaziniques indigoïdes, xanthéniques, phénanthridiniques, phtalocyanines, ceux dérivés du triarylméthane, les colorants naturels, seuls ou en mélanges.

7. Composition tinctoriale selon l'une quelconque des revendications 4 à 6, **caractérisée en ce que** le colorant direct représente de 0,001 à 20 % en poids par rapport au poids de la composition tinctoriale.

8. Composition tinctoriale selon l'une des revendications 4 à 7, **caractérisée en ce qu'**elle contient au moins une base d'oxydation et/ou au moins un coupleur.

9. Composition tinctoriale selon la revendication 8, **caractérisée en ce que** la base d'oxydation est choisie parmi les o-phénylènediamines, les p-phénylènediamines, les bases doubles, les o-aminophénols, les p-aminophénols, les bases hétérocycliques, leurs sels d'addition avec un acide, ainsi que leurs mélanges.

10. Composition tinctoriale selon l'une quelconque des revendications 4 à 9, **caractérisée en ce que** la base d'oxydation représente de 0,001 à 10 % en poids par rapport au poids de la composition tinctoriale.

11. Composition tinctoriale selon l'une quelconque des revendications 4 à 10, **caractérisée en ce que** le coupleur est choisi parmi les m-aminophénols, les m-phénylènediamines, les m-diphénols, les naphtols, les coupleurs hétérocycliques, leurs sels d'addition avec un acide, ainsi que leurs mélanges.

12. Composition tinctoriale selon l'une quelconque des revendications 4 à 11, **caractérisée en ce que** le coupleur représente de 0,001 à 10 % en poids par rapport au poids de la composition tinctoriale.

13. Composition tinctoriale selon l'une quelconque des revendications 4 à 12, **caractérisé en ce qu'**elle comprend au moins 40 % en poids d'eau par rapport au poids de la composition tinctoriale.

14. Composition tinctoriale selon l'une quelconque des revendications 4 à 13, **caractérisé en ce qu'**elle comprend au moins un tensioactif et/ou au moins un polymère.

15. Procédé de coloration de fibres kératiniques, dans lequel on met en oeuvre les étapes suivantes :
a) On applique sur les fibres kératiniques, une composition tinctoriale selon l'une quelconque des revendications 3 à 14 ;
b) On laisse poser pendant une durée suffisante pour obtenir l'effet souhaité,
c) on rince éventuellement les fibres kératiniques ;
d) on lave au shampooing et on rince les fibres, puis on les sèche ou on les laisse sécher.

16. Procédé de coloration de fibres kératiniques, dans lequel on met en oeuvre les étapes suivantes :
a) On applique simultanément ou successivement sur les fibres kératiniques, une composition tinctoriale selon l'une quelconque des revendications 3 à 14 et une composition oxydante comprenant au moins un agent oxydant ;
b) On laisse poser pendant une durée suffisante pour obtenir l'effet souhaité,
c) on rince éventuellement les fibres kératiniques ;
d) on lave au shampooing et on rince les fibres, puis on les sèche ou on les laisse sécher.

17. Procédé de coloration de fibres kératiniques, dans lequel on met en oeuvre les étapes suivantes :
a) On prépare une composition prête à l'emploi en mélangeant une composition colorante comprenant dans un milieu approprié à la coloration, au moins un colorant direct et/ou au moins un colorant d'oxydation, avec une composition comprenant au moins un agent oxydant; l'agent hydrotrope cationique tel que défini à l'une quelconque des revendications 1 à 3 se trouvant dans la composition colorante, dans la composition comprenant l'agent oxydant ou dans ces deux compositions simultanément,
b) On applique sur les fibres kératiniques, la composition prête à l'emploi ainsi obtenue et on laisse poser pendant une durée suffisante pour obtenir l'effet souhaité,
c) on rince éventuellement les fibres kératiniques ;
d) on lave au shampooing et on rince les fibres, puis on les sèche ou on les laisse sécher.

## Claims

1. Use of at least one direct dye and of at least one cationic hydrotrope of following formula (I): 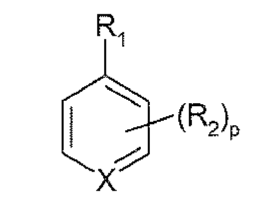 in which:
• X represents N⁺R₆ with R₆ representing a linear or branched C₁-C₁₀ alkyl radical, preferably a C₁-C₆ alkyl radical;
• R₁ represents:
- a hydrogen atom or a linear or branched C₁-C₁₀ alkyl radical, preferably a C₁-C₆ alkyl radical,
• R₂ represents a linear or branched C₁-C₄ alkyl radical;
• p has the value 0 or 1;
• the electrical neutrality of the compound being provided by a cosmetically acceptable anion or a mixture of cosmetically acceptable anions,
for the colouring of keratinous fibres.

2. Use according to Claim 1, **characterized in that** the cosmetically acceptable anion or the mixture of cosmetically acceptable anions is chosen from halides; sulphates; alkyl sulphates for which the linear or branched alkyl part is a C₁-C₆ alkyl part, such as the methosulphate or ethosulphate ions; hydrogencarbonates; perchlorates; salts of carboxylic acids, such as acetates, citrates or tartrates, alone or combined.

3. Use according to either one of Claims 1 and 2, **characterized in that** the cationic hydrotrope is chosen from N-butyl-3,4-dimethylpyridinium or N-butyl-4-methylpyridinium, alone or as mixtures.

4. Dyeing composition for colouring human keratinous fibres, comprising, in a medium appropriate for colouring, at least one cationic hydrotrope as defined in one of Claims 1 to 3 and at least one direct dye.

5. Dyeing composition according to the preceding claim, **characterized in that** the content of cationic hydrotrope is between 0.5 and 50% by weight, with respect to the weight of the dyeing composition, preferably between 0.5 and 20% by weight and more preferably still from 1 to 15% by weight.

6. Dyeing composition according to either one of Claims 4 and 5, **characterized in that** the direct dye is chosen from ionic or nonionic direct dyes, in particular of the type of the nitrobenzene dyes, azo, azomethine, methine, tetraazapentamethine, anthraquinone, naphthoquinone, benzoquinone, phenothiazine, indigoid, xanthene, phenanthridine or phthalocyanine dyes, those derived from triarylmethane or natural dyes, alone or as mixtures.

7. Dyeing composition according to any one of Claims 4 to 6, **characterized in that** the direct dye represents from 0.001 to 20% by weight, with respect to the weight of the dyeing composition.

8. Dyeing composition according to one of Claims 4 to 7, **characterized in that** it comprises at least one oxidation base and/or at least one coupler.

9. Dyeing composition according to Claim 8, **characterized in that** the oxidation base is chosen from o-phenylenediamines, p-phenylenediamines, double bases, o-aminophenols, p-aminophenols, heterocyclic bases, their addition salts with an acid and their mixtures.

10. Dyeing composition according to any one of Claims 4 to 9, **characterized in that** the oxidation base represents from 0.001 to 10% by weight, with respect to the weight of the dyeing composition.

11. Dyeing composition according to any one of Claims 4 to 10, **characterized in that** the coupler is chosen from m-aminophenols, m-phenylenediamines, m-diphenols, naphthols, heterocyclic couplers, their addition salts with an acid and their mixtures.

12. Dyeing composition according to any one of Claims 4 to 11, **characterized in that** the coupler represents from 0.001 to 10% by weight, with respect to the weight of the dyeing composition.

13. Dyeing composition according to any one of Claims 4 to 12, **characterized in that** it comprises at least 40% by weight of water, with respect to the weight of the dyeing composition.

14. Dyeing composition according to any one of Claims 4 to 13, **characterized in that** it comprises at least one surfactant and/or at least one polymer.

15. Process for colouring keratinous fibres, in which the following stages are carried out:
a) a dyeing composition according to any one of Claims 3 to 14 is applied to the keratinous fibres;
b) the composition is left to stand for a period of time sufficient to obtain the desired effect;
c) the keratinous fibres are optionally rinsed;
d) the fibres are washed with a shampoo and rinsed and then they are dried or left to dry.

16. Process for colouring keratinous fibres, in which the following stages are carried out:
a) a dyeing composition according to any one of Claims 3 to 14 and an oxidizing composition comprising at least one oxidizing agent are applied to the keratinous fibres, simultaneously or successively;
b) the mixture is left to stand for a period of time sufficient to obtain the desired effect;
c) the keratinous fibres are optionally rinsed;
d) the fibres are washed with a shampoo and rinsed and then they are dried or left to dry.

17. Process for colouring keratinous fibres, in which the following stages are carried out:
a) a ready-for-use composition is prepared by mixing a dye composition comprising, in a medium appropriate for colouring, at least one direct dye and/or at least one oxidation dye with a composition comprising at least one oxidizing agent; the cationic hydrotropic agent as defined in any one of Claims 1 to 3 occurring in the dye composition, in the composition comprising the oxidizing agent or in both these compositions simultaneously,
b) the ready-for-use composition thus obtained is applied to the keratinous fibres and is left to stand for a period of time sufficient to obtain the desired effect;
c) the keratinous fibres are optionally rinsed;
d) the fibres are washed with a shampoo and rinsed and then they are dried or left to dry.

## Patentansprüche

1. Verwendung mindestens eines Direktfarbstoffs und mindestens eines kationischen Hydrotropikums der folgenden Formel (I) : 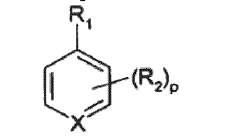 worin:
• X für N⁺R₆ steht, wobei R₆ für einen linearen oder verzweigten C₁-C₁₀-Alkylrest, vorzugsweise C₁-C₆-Alkylrest, steht;
• R₁ für ein Wasserstoffatom oder einen linearen oder verzweigten C₁-C₁₀-Alkylrest, vorzugsweise C₁-C₆-Alkylrest, steht;
• R₂ für einen linearen oder verzweigten C₁-C₄-Alkylrest steht;
• p den Wert 0 oder 1 aufweist;
• wobei die Elektroneutralität der Verbindung durch ein kosmetisch unbedenkliches Anion oder eine Mischung von kosmetisch unbedenklichen Anionen gewährleistet ist;
zum Färben von Keratinfasern.

2. Verwendung nach Anspruch 1, **dadurch gekennzeichnet, dass** das kosmetisch unbedenkliche Anion oder die Mischung von kosmetisch unbedenklichen Anionen aus Halogeniden; Sulfaten; Alkylsulfaten mit 1-6 C-Atomen im linearen oder verzweigten Alkylteil,
wie Methosulfat- oder Ethosulfationen; Hydrogencarbonaten; Perchloraten; Carbonsäuresalzen wie Acetaten, Citraten und Tartaten, alleine oder kombiniert, ausgewählt ist.

3. Verwendung nach einem der Ansprüche 1 und 2, **dadurch gekennzeichnet, dass** das kationische Hydrotropikum aus N-Butyl-3,4-dimethylpyridinium-und N-Butyl-4-methylpyridiniumsalzen, alleine oder als Mischungen, ausgewählt ist.

4. Färbezusammensetzung zum Färben von menschlichen Keratinfasern, die in einem für die Färbung geeigneten Medium mindestens ein kationisches Hydrotropikum gemäß einem der Ansprüche 1 bis 3 und mindestens einen Direktfarbstoff umfasst.

5. Färbezusammensetzung nach dem vorhergehenden Anspruch, **dadurch gekennzeichnet, dass** der Gehalt an kationischem Hydrotropikum zwischen 0,5 und 50 Gew.-%, bezogen auf das Gewicht der Färbezusammensetzung, und vorzugsweise zwischen 0,5 und 20 Gew.-% liegt und noch weiter bevorzugt 1 bis 15 Gew.-% beträgt.

6. Färbezusammensetzung nach einem der Ansprüche 4 oder 5, **dadurch gekennzeichnet, dass** der Direktfarbstoff aus ionischen oder nichtionischen Direktfarbstoffen, insbesondere vom Typ der Nitrobenzol-, Azo-, Azomethin-, Methin-, Tetraazapentamethin-, Anthrachinon-, Naphthochinon-, Benzochinon-, Phenothiazin-, Indigoid-, Xanthen-, Phenanthridin- und Phthalocyanin-Farbstoffe, von Triarylmethan abgeleiteten Farbstoffe und natürlichen Farbstoffe, alleine oder als Mischungen, ausgewählt ist.

7. Färbezusammensetzung nach einem der Ansprüche 4 bis 6, **dadurch gekennzeichnet, dass** der Direktfarbstoff 0,001 bis 20 Gew.-%, bezogen auf das Gewicht der Färbezusammensetzung, ausmacht.

8. Färbezusammensetzung nach einem der Ansprüche 4 bis 7, **dadurch gekennzeichnet, dass** sie mindestens eine Oxidationsbase und/oder mindestens einen Kuppler enthält.

9. Färbezusammensetzung nach Anspruch 8, **dadurch gekennzeichnet, dass** die Oxidationsbase aus o-Phenylendiaminen, p-Phenylendiaminen, Doppelbasen, o-Aminophenolen, p-Aminophenolen, heterocyclischen Basen, Additionssalzen davon mit einer Säure sowie Mischungen davon ausgewählt ist.

10. Färbezusammensetzung nach einem der Ansprüche 4 bis 9, **dadurch gekennzeichnet, dass** die Oxidationsbase 0,001 bis 10 Gew.-%, bezogen auf das Gewicht der Färbezusammensetzung, ausmacht.

11. Färbezusammensetzung nach einem der Ansprüche 4 bis 10, **dadurch gekennzeichnet, dass** der Kuppler aus m-Aminophenolen, m-Phenylendiaminen, m-Diphenolen, Naphtholen, heterocyclischen Kupplern, Additionssalzen davon mit einer Säure sowie Mischungen davon ausgewählt ist.

12. Färbezusammensetzung nach einem der Ansprüche 4 bis 11, **dadurch gekennzeichnet, dass** der Kuppler 0,001 bis 10 Gew.-%, bezogen auf das Gewicht der Färbezusammensetzung, ausmacht.

13. Färbezusammensetzung nach einem der Ansprüche 4 bis 12, **dadurch gekennzeichnet, dass** sie mindestens 40 Gew.-% Wasser, bezogen auf das Gewicht der Färbezusammensetzung, umfasst.

14. Färbezusammensetzung nach einem der Ansprüche 4 bis 13, **dadurch gekennzeichnet, dass** sie mindestens ein Tensid und/oder mindestens ein Polymer umfasst.

15. Verfahren zum Färben von Keratinfasern, bei dem man:
a) auf die Keratinfasern eine Färbezusammensetzung nach einem der Ansprüche 3 bis 14 aufbringt;
b) über einen zur Erzielung des gewünschten Effekts ausreichenden Zeitraum einwirken lässt;
c) die Keratinfasern gegebenenfalls spült;
d) die Fasern mit einem Shampoo wäscht und spült und dann trocknet oder trocknen lässt.

16. Verfahren zum Färben von Keratinfasern, bei dem man:
a) auf die Keratinfasern gleichzeitig oder nacheinander eine Färbezusammensetzung nach einem der Ansprüche 3 bis 14 und eine oxidierend wirkende Zusammensetzung, die mindestens ein Oxidationsmittel umfasst, aufbringt;
b) über einen zur Erzielung des gewünschten Effekts ausreichenden Zeitraum einwirken lässt;
c) die Keratinfasern gegebenenfalls spült;
d) die Fasern mit einem Shampoo wäscht und spült und dann trocknet oder trocknen lässt.

17. Verfahren zum Färben von Keratinfasern, bei dem man:
a) durch Mischen einer Färbezusammensetzung, die in einem für die Färbung geeigneten Medium mindestens einen Direktfarbstoff und/oder mindestens einen Oxidationsfarbstoff umfasst, mit einer Zusammensetzung, die mindestens ein Oixidationsmittel umfasst, eine gebrauchsfertige Zusammensetzung herstellt; wobei das kationische hydrotrope Mittel gemäß einem der Ansprüche 1 bis 3 in der Färbezusammensetzung, in der Zusammensetzung, die das Oxidationsmittel umfasst, oder in diesen beiden Zusammensetzungen gleichzeitig vorliegt,
b) die so erhaltene gebrauchsfertige Zusammensetzung auf die Keratinfasern aufbringt und über einen zur Erzielung des gewünschten Effekts ausreichenden Zeitraum einwirken lässt;
c) die Keratinfasern gegebenenfalls spült;
d) die Fasern mit einem Shampoo wäscht und spült und dann trocknet oder trocknen lässt.
